# EUROPEAN PATENT APPLICATION

(11) **EP 4 834 127 A1**
(43) Date of publication of application: **04.11.2026**
(21) Application number: 24913061.8
(22) Date of filing: 26.12.2024
(51) Int. Cl.: A23L 33/105, A61K 8/9761, A61K 8/9767, A61K 8/9789, A61K 8/9794, A61K 36/14, A61K 36/15, A61K 36/67, A61K 36/73, A61K 36/74, A61K 36/9066, A61P 1/00, A61P 3/00, A61P 15/00, A61P 17/00, A61P 17/14, A61P 25/00, A61P 25/28, A61P 43/00, A61Q 5/00, A61Q 19/00

(54) **ALTERNATIVE AUTOPHAGY ACTIVATOR**

(30) Priority: 26.12.2023 JP 2023219968
(71) Applicant: Ezaki Glico Co., Ltd., Osaka-shi, Osaka 555-8502 (JP)
(72) Inventor: IKEHARA Ryohei, Osaka-shi, Osaka 555-8502 (JP); NISHIURA Hiromi, Osaka-shi, Osaka 555-8502 (JP); INOOKA Hiroshi, Osaka-shi, Osaka 555-8502 (JP); SAMBE Haruyo, Osaka-shi, Osaka 555-8502 (JP); FUKUDA Junya, Osaka-shi, Osaka 555-8502 (JP); SUZUKI Daisuke, Osaka-shi, Osaka 555-8502 (JP); NIBE Yoichi, Tokyo 113-8510 (JP); SHIMIZU Shigeomi, Tokyo 113-8510 (JP)
(74) Representative: D Young & Co LLP
(86) International application number: PCT/JP2024/046289
(87) International publication number: WO 2025/143175

(57) **Abstract**

An alternative autophagy activator is provided, which contains at least one selected from the group consisting of a coffee extract and/or a coffee ground extract, a black pepper extract, a Japanese red pine extract, a juniper berry extract, a turmeric extract, and an apple polyphenol extract.

## Description

### Technical Field

The present invention relates to an alternative autophagy activator. The present invention also relates to a food or beverage composition for activating alternative autophagy.

### Background Art

Autophagy is known as an intracellular purifying and recycling system equipped in all eukaryotic organisms, including yeast and plants, and studies thereon have been progressing in recent years.

As studies on the molecular mechanisms of autophagy progress, over 30 autophagy-related molecules have been identified, and it is considered that, among these molecules, particularly Atg5, Atg7, LC3, and the like are related to autophagy and are essential molecules for the expression of the action of autophagy.

However, nowadays, the presence of novel autophagy has been discovered that does not require these molecules, originates from the Golgi apparatus or endosomes, and is regulated by molecules such as Rab9 (Non-Patent Literature 1). Such autophagy is called alternative autophagy to distinguish this from conventional autophagy that is dependent on Atg5, Atg7, LC3, and the like (sometimes simply referred to as Atg5-dependent autophagy).

In recent years, it has become clear that a decrease in the activity of alternative autophagy leads to a decline in the function of cells and tissues, and Non-Patent Literature 2 discloses that enteritis worsens in mice lacking Trim31, i.e., an alternative autophagy-related protein.

In addition, Patent Literature 1 discloses an alternative autophagy inducer and an anticancer agent that contain a benzothiophene compound as an active ingredient. Patent Literature 2 discloses an inhibitor of ultraviolet light-induced inflammation, which contains an alternative autophagy inducer as an active ingredient.

### Citation List

### Patent Literature

Patent Literature 1: WO 2013/118842
Patent Literature 2: WO 2020/091070

### Non-Patent Literature

Non-Patent Literature 1: Nishida et al., Nature, 2009, 641, 654-658
Non-Patent Literature 2: Yoichi Nibe et al., Research Report by Grant-in-Aid for Scientific Research, 2021, Research Topic/Area Number 19K17426

### Summary of Invention

### Technical Problem

An object to be achieved by the present invention is to provide a novel component capable of activating alternative autophagy.

### Solution to Problem

The present inventors have conducted extensive investigation to achieve the object, and as a result, have discovered that a coffee ground extract, a coffee extract, a black pepper extract, a Japanese red pine extract, a juniper berry extract, a turmeric extract or an apple polyphenol extract activates alternative autophagy, and thus completed the present invention.

The present invention is as follows.
[1] An alternative autophagy activator containing at least one selected from the group consisting of a coffee ground extract, a coffee extract, a black pepper extract, a Japanese red pine extract, a juniper berry extract, a turmeric extract, and an apple polyphenol extract.
[2] The alternative autophagy activator according to [1], which activates alternative autophagy as at least one selected from the group consisting of a coffee ground extract, a coffee extract, a black pepper extract, a Japanese red pine extract, a juniper berry extract, a turmeric extract, and an apple polyphenol extract promotes fusion of a lysosomal protein with an autophagosome independently of Atg5, Atg7, and/or LC3.
[3] The alternative autophagy activator according to [1] or [2], which is used for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[4] A food or beverage composition for activating alternative autophagy, the food or beverage composition containing at least one selected from the group consisting of a coffee ground extract, a coffee extract, a black pepper extract, a Japanese red pine extract, a juniper berry extract, a turmeric extract, and an apple polyphenol extract.
[5] A food or beverage composition for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality, the food or beverage composition containing at least one selected from the group consisting of a coffee ground extract, a coffee extract, a black pepper extract, a Japanese red pine extract, a juniper berry extract, a turmeric extract, and an apple polyphenol extract.
[6] The food or beverage composition according to [5], which exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy by at least one selected from the group consisting of a coffee ground extract, a coffee extract, a black pepper extract, a Japanese red pine extract, a juniper berry extract, a turmeric extract, and an apple polyphenol extract.
[7] The food or beverage composition according to [5] or [6], which exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality as at least one selected from the group consisting of a coffee ground extract, a coffee extract, a black pepper extract, a Japanese red pine extract, a juniper berry extract, a turmeric extract, and an apple polyphenol extract promotes fusion of a lysosomal protein with an autophagosome independently of Atg5, Atg7, and/or LC3.

The present invention may be as follows.
[1A]
   An alternative autophagy activator containing at least one selected from the group consisting of dehydrocafestol (DC) and dehydrokahweol (DK).
[2A]
   The alternative autophagy activator according to [1A], which activates alternative autophagy as at least one selected from the group consisting of dehydrocafestol (DC) and dehydrokahweol (DK) promotes fusion of a lysosomal protein with an autophagosome independently of Atg5, Atg7, and/or LC3.
[3A]
   The alternative autophagy activator according to [1A] or [2A], which is used for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[4A]
   A food or beverage composition for activating alternative autophagy, the food or beverage composition containing at least one selected from the group consisting of dehydrocafestol (DC) and dehydrokahweol (DK).
[5A]
   A food or beverage composition for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality, the food or beverage composition containing at least one selected from the group consisting of dehydrocafestol (DC) and dehydrokahweol (DK).
[6A]
   The food or beverage composition according to [5A], which exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy by at least one selected from the group consisting of dehydrocafestol (DC) and dehydrokahweol (DK).
[7A]
   The food or beverage composition according to [5A] or [6A], which exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality as at least one selected from the group consisting of dehydrocafestol (DC) and dehydrokahweol (DK) promotes fusion of a lysosomal protein with an autophagosome independently of Atg5, Atg7, and/or LC3.

In the respective aspects [1] to [7], the coffee ground extract may contain DC and/or DK, and the DC and DK may be derived from the coffee ground extract.
[A1]
   A method for activating alternative autophagy using at least one selected from the group consisting of a coffee ground extract, a coffee extract, a black pepper extract, a Japanese red pine extract, a juniper berry extract, a turmeric extract, and an apple polyphenol extract.
[A2]
   The method according to [A1], in which alternative autophagy is activated by promoting fusion of a lysosomal protein with an autophagosome independently of Atg5, Atg7, and/or LC3 using at least one selected from the group consisting of a coffee ground extract, a coffee extract, a black pepper extract, a Japanese red pine extract, a juniper berry extract, a turmeric extract, and an apple polyphenol extract.
[A3]
   The method according to [A1] or [A2], in which alternative autophagy is activated for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[A4]
   A method for activating alternative autophagy using a food or beverage composition containing at least one selected from the group consisting of a coffee ground extract, a coffee extract, a black pepper extract, a Japanese red pine extract, a juniper berry extract, a turmeric extract, and an apple polyphenol extract.
[A5]
   A method for bringing about at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality using a food or beverage composition containing at least one selected from the group consisting of a coffee ground extract, a coffee extract, a black pepper extract, a Japanese red pine extract, a juniper berry extract, a turmeric extract, and an apple polyphenol extract.
[A6]
   The method according to [A5], in which at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality is brought about by activating alternative autophagy using a food or beverage composition containing at least one selected from the group consisting of a coffee ground extract, a coffee extract, a black pepper extract, a Japanese red pine extract, a juniper berry extract, a turmeric extract, and an apple polyphenol extract.
[A7]
   The method according to [A5] or [A6], in which at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality is brought about by promoting fusion of a lysosomal protein with an autophagosome independently of Atg5, Atg7, and/or LC3 using a food or beverage composition containing at least one selected from the group consisting of a coffee ground extract, a coffee extract, a black pepper extract, a Japanese red pine extract, a juniper berry extract, a turmeric extract, and an apple polyphenol extract.
[B1]
   Use of at least one selected from the group consisting of a coffee ground extract, a coffee extract, a black pepper extract, a Japanese red pine extract, a juniper berry extract, a turmeric extract, and an apple polyphenol extract for activating alternative autophagy.
[B2]
   The use according to [B1], which is for activating alternative autophagy by promoting fusion of a lysosomal protein with an autophagosome independently of Atg5, Atg7, and/or LC3.
[B3]
   The use according to [B1] or [B2], which is for activating alternative autophagy for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[B4]
   Use of a food or beverage composition containing at least one selected from the group consisting of a coffee ground extract, a coffee extract, a black pepper extract, a Japanese red pine extract, a juniper berry extract, a turmeric extract, and an apple polyphenol extract for activating alternative autophagy.
[B5]
   Use of a food or beverage composition containing at least one selected from the group consisting of a coffee ground extract, a coffee extract, a black pepper extract, a Japanese red pine extract, a juniper berry extract, a turmeric extract, and an apple polyphenol extract for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[B6]
   The use according to [B5], which is for bringing about at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy by at least one selected from the group consisting of a coffee ground extract, a coffee extract, a black pepper extract, a Japanese red pine extract, a juniper berry extract, a turmeric extract, and an apple polyphenol extract.
[B7]
   The use according to [B5] or [B6], which is for bringing about at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by promotion of fusion of a lysosomal protein with an autophagosome independently of Atg5, Atg7, and/or LC3 by at least one selected from the group consisting of a coffee ground extract, a coffee extract, a black pepper extract, a Japanese red pine extract, a juniper berry extract, a turmeric extract, and an apple polyphenol extract.
[C1]
   Use of at least one selected from the group consisting of a coffee ground extract, a coffee extract, a black pepper extract, a Japanese red pine extract, a juniper berry extract, a turmeric extract, and an apple polyphenol extract in the manufacture of an alternative autophagy activator.
[C2]
   The use according to [C1], in the manufacture of an alternative autophagy activator that activates alternative autophagy by promoting fusion of a lysosomal protein with an autophagosome independently of Atg5, Atg7, and/or LC3.
[C3]
   The use according to [C1] or [C2], in the manufacture of an alternative autophagy activator that activates alternative autophagy for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[C4]
   Use of at least one selected from the group consisting of a coffee ground extract, a coffee extract, a black pepper extract, a Japanese red pine extract, a juniper berry extract, a turmeric extract, and an apple polyphenol extract in the manufacture of a food or beverage composition for activating alternative autophagy.
[C5]
   Use of at least one selected from the group consisting of a coffee ground extract, a coffee extract, a black pepper extract, a Japanese red pine extract, a juniper berry extract, a turmeric extract, and an apple polyphenol extract in the manufacture of a food or beverage composition for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[C6]
   The use according to [C5], in the manufacture of a food or beverage composition that exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy by at least one selected from the group consisting of a coffee ground extract, a coffee extract, a black pepper extract, a Japanese red pine extract, a juniper berry extract, a turmeric extract, and an apple polyphenol extract.
[C7]
   The use according to [C5] or [C6], in the manufacture of a food or beverage composition that exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality as at least one selected from the group consisting of a coffee ground extract, a coffee extract, a black pepper extract, a Japanese red pine extract, a juniper berry extract, a turmeric extract, and an apple polyphenol extract promotes fusion of a lysosomal protein with an autophagosome independently of Atg5, Atg7, and/or LC3.
[D1]
   A coffee ground extract, a coffee extract, a black pepper extract, a Japanese red pine extract, a juniper berry extract, a turmeric extract, and an apple polyphenol extract, for use in activating alternative autophagy.
[D2]
   The coffee ground extract, the coffee extract, the black pepper extract, the Japanese red pine extract, the juniper berry extract, the turmeric extract, and the apple polyphenol extract according to [D1], for use in activating alternative autophagy by promoting fusion of a lysosomal protein with an autophagosome independently of Atg5, Atg7, and/or LC3.
[D3]
   The coffee ground extract, the coffee extract, the black pepper extract, the Japanese red pine extract, the juniper berry extract, the turmeric extract, and the apple polyphenol extract according to [D1] or [D2], for use in activating alternative autophagy for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[D4]
   A food or beverage composition containing at least one selected from the group consisting of a coffee ground extract, a coffee extract, a black pepper extract, a Japanese red pine extract, a juniper berry extract, a turmeric extract, and an apple polyphenol extract, for use in activating alternative autophagy.
[D5]
   A food or beverage composition containing at least one selected from the group consisting of a coffee ground extract, a coffee extract, a black pepper extract, a Japanese red pine extract, a juniper berry extract, a turmeric extract, and an apple polyphenol extract, which is used for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.
[D6]
   The food or beverage composition according to [D5], which is used for bringing about at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy by at least one selected from the group consisting of a coffee ground extract, a coffee extract, a black pepper extract, a Japanese red pine extract, a juniper berry extract, a turmeric extract, and an apple polyphenol extract.
[D7]
   The food or beverage composition according to [D5] or [D6], for use in bringing about at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by promotion of fusion of a lysosomal protein with an autophagosome independently of Atg5, Atg7, and/or LC3 by at least one selected from the group consisting of a coffee ground extract, a coffee extract, a black pepper extract, a Japanese red pine extract, a juniper berry extract, a turmeric extract, and an apple polyphenol extract.

In [A1] to [D7] above, at least one selected from the group consisting of dehydrocafestol (DC) and dehydrokahweol (DK) or a food or beverage composition containing at least one selected from the group consisting of dehydrocafestol (DC) and dehydrokahweol (DK) may be used as an active ingredient instead of the prescribed extract, and the coffee ground extract may contain DC and/or DK.

In the present invention, particularly in [A1] to [D7] above, the invention may be a therapeutic invention but may be a non-therapeutic invention. In other words, the invention may be an invention of a therapeutic or non-therapeutic method, an invention of a therapeutic or non-therapeutic use, or an invention of a therapeutic or non-therapeutic substance.

In the present invention, a "non-therapeutic invention" means an invention that does not involve medical practice in a case of administered to a subject and a case of ingested by a subject, that is, an invention that does not involve a method for operating, treating, or diagnosing a subject, especially a human. In some cases, the subject may also include a non-human animal. More specifically, a "non-therapeutic invention" means an invention that does not involve a method for performing surgery, treatment, or diagnosis on a human by a physician or a person under the direction of a physician.

Examples of the "non-therapeutic invention" include an invention aimed at health promotion or an invention aimed at beauty. The "non-therapeutic invention" may be a "preventive invention" as long as it does not fall under the category of a "therapeutic invention."

### Advantageous Effect of Invention

According to the present invention, it is possible to provide a novel component capable of activating alternative autophagy.

### Brief Description of Drawings

[Figure 1] Figure 1 illustrates results of evaluation of alternative autophagy activity.
[Figure 2] Figure 2 illustrates results of evaluation of alternative autophagy activity.

### Description of Embodiments

Hereinafter, an embodiment of the present invention (hereinafter referred to as "the present embodiment") will be described, but the scope of the present invention should not be interpreted as being limited to the following embodiment.

An embodiment of the present invention relates to an alternative autophagy activator containing at least one selected from the group consisting of a coffee ground extract, a coffee extract, a black pepper extract, a Japanese red pine extract, a juniper berry extract, a turmeric extract, and an apple polyphenol extract.

As used herein, an extract refers to a composition containing a particular component separated from a raw material, and a food or beverage composition containing an extract is distinct from a raw material itself.

In the present embodiment, for the preparation of an extract (including a fraction), any methods and devices known to those skilled in the art may be used, and examples of the preparation method include a preparation method by solvent extraction, supercritical extraction, steam distillation, pressing or the like. An extract (including a fraction) may be further prepared from an extract (may be a fraction (including a pressing)) obtained by pressing. When an extract (including a fraction) is prepared, in solvent extraction among others, a solvent may be appropriately used depending on the raw material and the particular component to be acquired, and examples of the solvent include water, acetic acid, ethanol, methanol, acetone, hexane, benzene, or diethyl ether. The solvent used for extraction from coffee grounds is preferably a solvent containing an organic solvent.

The form of the obtained extract (including a fraction) may be, for example, any form such as a liquid, solid, or powder. The form in a case of a liquid may be a liquid in the solvent used for extraction, or may be, for example, a liquid concentrate obtained by concentration. The form may also be a suspension in which a portion of the extract (including a fraction) is precipitated during the concentration process.

The liquid extract (including a fraction) may be subjected to a drying treatment such as freeze drying or spray drying either directly or together with an excipient to obtain a solid extract (including a fraction).

As used herein, "coffee" refers to the seeds (coffee beans) of a plant belonging to the genus Coffea. In the present embodiment, only one species of the genus Coffea may be used or two or more species may be used in combination.

As used herein, "black pepper" refers to a plant of which the scientific name is Piper nigrum, among climbing plants belonging to the genus Piper.

As used herein, "Japanese red pine" refers to a coniferous tree of which the scientific name is Pinus densiflora, among evergreen coniferous trees belonging to the genus Pinus.

As used herein, "juniper berry" refers to the fruit of Juniperus communis that is a coniferous tree belonging to the genus Juniperus.

As used herein, "turmeric" refers to a perennial plant belonging to the genus Curcuma, and is sometimes called Curcuma longa. Examples thereof include C. longa, C. aromatica, C. xanthorrhiza, and C. zedoaria. "Turmeric" is preferably C. longa. In the present embodiment, only one species of the genus Curcuma may be used or two or more species may be used in combination.

As used herein, "apple" refers to the fruit of a deciduous tree belonging to the genus Malus. In the present embodiment, only one species of the genus Malus may be used or two or more species may be used in combination.

As used herein, the "coffee ground extract" refers to a component separated by extraction from coffee grounds after extraction with water or the like, and the "coffee extract" refers to a component separated by extraction from coffee beans (ground coffee beans, and the like; the same applies hereinafter).

The coffee ground extract may be, for example, an ethanol or acetone extract of grounds obtained by extracting coffee beans with water or the like (also called coffee extraction grounds). The concentration of the solvent used for extraction is not particularly limited, but may be 50% to 100%, 70% to 100%, 70% to 99.5%, or 80% to 99.5%. The coffee ground extract may be, for example, a pressing of grounds obtained by extracting coffee beans with water or the like (coffee ground pressing), or may be coffee oil derived from coffee extraction grounds. An alcohol-soluble component of coffee ground pressing may be used as a coffee ground extract. Examples of the alcohol include methanol and ethanol.

The coffee extract and/or coffee ground extract may be a mixture of a coffee extract and a coffee ground extract.

The coffee beans may be green beans (including beans subjected to fermentation treatment; the same applies hereinafter), or roasted beans, or beans including both green beans and roasted beans. As the coffee beans, green coffee beans may be directly ground into powder or roasted and ground into powder. The coffee beans may consist of one kind of coffee beans or two or more kinds of beans.

The coffee extract or coffee ground extract may contain dehydrocafestol (DC) and/or dehydrokahweol (DK). As used here, the invention related to a coffee extract or coffee ground extract may be understood as an invention in which the coffee extract or coffee ground extract is read as dehydrocafestol (DC) and/or dehydrokahweol (DK).

The structure of DC is presented below.

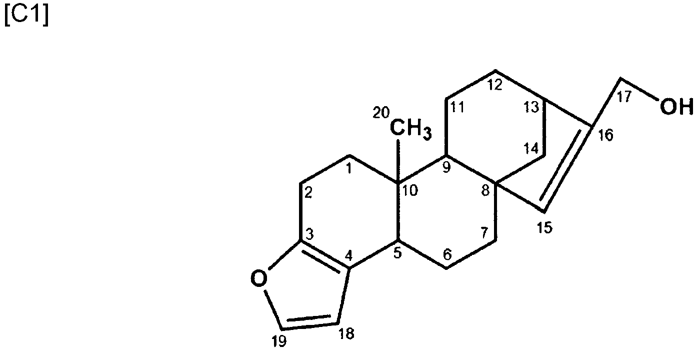

The structure of DK is presented below.

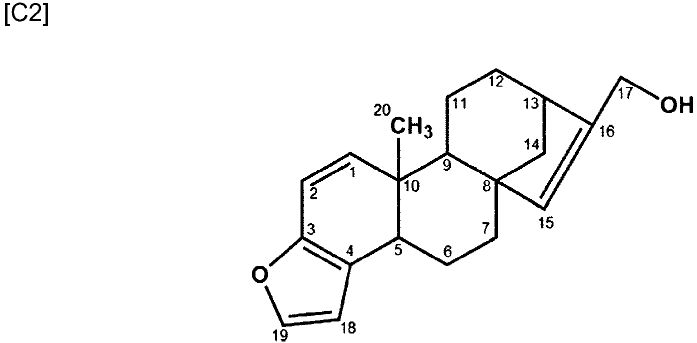

The method for obtaining a coffee ground extract containing DC and/or DK is not particularly limited. The method described below for obtaining a coffee ground extract containing DC and/or DK can also be utilized as a method for obtaining a coffee ground extract.

The extraction solvent for obtaining coffee grounds is preferably water, and the extraction temperature may be, for example, 70°C or more or a low temperature of about 5°C to 40°C.

The coffee beans used are preferably roasted coffee beans. The roasting conditions can be determined appropriately depending on the kind of coffee beans and the like, but for example, the coffee beans may be roasted for 8 minutes or more at a temperature of 200°C or more. For example, as roasted (preferably dark roasted) coffee beans (or crushed product thereof), commercially available ones may be used.

The obtained coffee grounds may be used as they are for the preparation of coffee ground extract. The coffee grounds may be further dried from the viewpoint of obtaining a coffee ground extract containing DC and/or DK at higher concentrations. As the drying method, drying by air blowing, dry heat drying, vacuum drying, freeze drying, or the like may be used.

After coffee grounds are obtained, a coffee ground extract may be prepared using methods and devices known to those skilled in the art, and may be prepared by, for example, solvent extraction, and a solvent containing an organic solvent may be used. The obtained coffee ground extract preferably contains DC and/or DK.

Specifically, the coffee ground extract containing DC and/or DK may be produced as follows.

Coffee beans including Arabica coffee beans are roasted at 220°C to 260°C for 8 minutes or more. The roasted coffee beans are crushed using a coffee mill, and then water at 75°C to 95°C is dripped onto the crushed coffee beans using a filter to obtain coffee grounds. The obtained coffee grounds are dried at less than 100°C for 5 to 20 hours until the moisture value is 10% or less. To the dried coffee grounds, 70% to 95% (v/v) ethanol is added, and the mixture is stirred at 40°C to 60°C for 1 to 6 hours and then filtered using filter paper to recover the filtrate. The same procedure as above is carried out on the coffee grounds remaining on the filter paper to recover the filtrate, and this process is repeated 1 to 3 times. The recovered filtrates are collected, and the combined filtrate is concentrated using an evaporator until ethanol is removed to obtain a coffee ground extract containing DC and/or DK.

DC and DK may be isolated from coffee grounds or the like as a raw material, or may be commercially available products.

The method for isolating DC and DK from coffee grounds is not particularly limited, and can be isolated from, for example, extracts or pressings of coffee grounds by chromatography or the like. In a case where DC and DK are isolated by chromatography, a solvent such as water, acetonitrile, or alcohols can be used as the mobile phase, and one kind of solvent may be used, or a mixed solvent of two or more kinds of solvents may be used, and elution may be performed with a mobile phase under gradient conditions.

DC and DK can be isolated, for example, from coffee grounds as a raw material as follows.

Coffee grounds having a moisture value of 10% or less are obtained by the method described above. The obtained coffee grounds are heated to 80°C to 100°C using a roasting kettle, and pressing is performed on the heated coffee grounds one to three times to recover oil. The recovered oil is filtered to obtain coffee ground pressed oil.

Hexane in an amount 2 to 6 times the amount of the coffee ground pressed oil is added and mixed, and then methanol in an amount 1 to 3 times the amount of the coffee ground pressed oil is added and mixed. After the mixture is left to still stand for a while and confirmed to be separated into two layers, the methanol layer is recovered. Methanol in an amount equal to that of the coffee ground pressed oil is again added to and mixed with the recovered methanol layer. The procedure is repeated 1 to 3 times to obtain a methanol layer. Distilled water in an amount 1/5 to 1/15 times the amount of the coffee ground pressed oil is added to and mixed with this methanol layer, the mixture is left to still stand for separation, and then the methanol layer is recovered. The recovered methanol layer is concentrated to dryness to obtain a dried product, and the dried product thus obtained is then subjected to reverse phase chromatography for fractionation. Using ultrapure water, acetonitrile, and isopropanol as mobile phases, gradient elution is performed from ultrapure water-acetonitrile (95:5) to 100% acetonitrile, then isopropanol is poured, and the eluted fraction is concentrated to dryness to obtain a dried product. The dried product is subjected to HPLC to isolate each of DC and DK. Using ultrapure water and acetonitrile as mobile phases, ultrapure water-acetonitrile (60:40) is allowed to flow and the eluate for each peak is fractionated. The component corresponding to each peak is identified, and the eluates identified as DC and DK are designated as DC and DK isolates, respectively.

As used herein, a black pepper extract, a Japanese red pine extract, a juniper berry extract, or a turmeric extract refers to a component separated by extraction from black pepper, Japanese red pine, juniper berry, or turmeric as a raw material.

The plant as a raw material for each extract may be the plant itself, a dried product of the plant, a cut or shredded product of the plant itself or a dried product of the plant, or a powder.

As used herein, each extract may be, for example, as follows.

The Japanese red pine extract may be an ethanol extract of Japanese red pine or an ethanol extract of Japanese red pine powder. The concentration of ethanol used for extraction is not particularly limited, but may be 50% to 100%, 70% to 100%, 70% to 99.5%, or 80% to 99.5%.

The juniper berry extract may be an ethanol extract of juniper berries or an ethanol extract of juniper berry powder. The concentration of ethanol used for extraction is not particularly limited, but may be 50% to 100%, 70% to 100%, 70% to 99.5%, or 80% to 99.5%.

The apple polyphenol extract may be a fraction containing apple polyphenols (an apple polyphenol fraction derived from apples).

The black pepper extract may be an ethanol extract of black pepper or an ethanol extract of black pepper powder. The concentration of ethanol used for extraction is not particularly limited, but may be 50% to 100%, 70% to 100%, 70% to 99.5%, or 80% to 99.5%. The black pepper extract may contain piperine as a main component.

In the present embodiment, as each of the coffee ground extract, the coffee extract, the black pepper extract, the Japanese red pine extract, the juniper berry extract, the turmeric extract, and the apple polyphenol extract, a commercially available one may be used.

As each of the black pepper extract or turmeric extract, for example, a commercially available product containing piperine or curcumin as a main component may be used. The prepared extract may also be mixed with a commercially available product and used as an extract (including a fraction).

Specific examples of the turmeric extract include products that contain three compounds (curcuminoids): curcumin, demethoxycurcumin (DMC), and bisdemethoxycurcumin (BDMC), and are standardized to 95% or more curcuminoids. The turmeric extract may be a product derived from dried Curcuma longa rhizomes.

As the apple polyphenol extract, a commercially available product containing apple polyphenols as a main component may be used.

The alternative autophagy activator in the present embodiment may contain at least one selected from the group consisting of a coffee ground extract, a coffee extract, a black pepper extract, a Japanese red pine extract, a juniper berry extract, a turmeric extract, and an apple polyphenol extract in an arbitrary amount as long as it can exert an alternative autophagy activating action. The alternative autophagy activator may contain other active ingredients as long as the action thereof is not impaired. The other active ingredients may be other components known as an alternative autophagy activator or may be other components that are not known to have or does not have the alternative autophagy activating ability.

The alternative autophagy activator may contain only at least one or only two or more selected from the group consisting of a coffee ground extract, a coffee extract, a black pepper extract, a Japanese red pine extract, a juniper berry extract, a turmeric extract, and an apple polyphenol extract.

The alternative autophagy activator in the present embodiment contains at least one selected from the group consisting of a coffee ground extract, a coffee extract, a black pepper extract, a Japanese red pine extract, a juniper berry extract, a turmeric extract, and an apple polyphenol extract and thus is capable of activating alternative autophagy.

In the present embodiment, the activation of alternative autophagy may induce or promote the degradation of intracellular molecules such as proteins, organelles, and bacteria and viruses that invade from outside the cell, for example independently of Atg5, Atg7, and/or LC3. The activation of alternative autophagy may be induced by fusion of lysosomal proteins with autophagosomes independently of Atg5, Atg7, and/or LC3. In other words, the activation of alternative autophagy may be induced by the fusion of lysosomal proteins with autophagosomes independently of at least one molecule selected from the group consisting of Atg5, Atg7, and LC3, or may be induced by the fusion of lysosomal proteins with autophagosomes independently of all the molecules of Atg5, Atg7, and LC3.

The alternative autophagy activator in the present embodiment may be an alternative autophagy activator which activates alternative autophagy as at least one selected from the group consisting of a coffee ground extract, a coffee extract, a black pepper extract, a Japanese red pine extract, a juniper berry extract, a turmeric extract, and an apple polyphenol extract promotes the fusion of lysosomal proteins with autophagosomes independently of Atg5, Atg7, and/or LC3 or independently of at least one molecule selected from the group consisting of Atg5, Atg7, and LC3.

In the present embodiment, the activation of alternative autophagy may induce or promote the fusion of lysosomal proteins with autophagosomes independently of autophagy-related molecules, including Atg5, Atg7, and LC3, and may induce or promote the degradation of intracellular molecules such as proteins, organelles, and bacteria and viruses that invade from outside the cell. In other words, the activation of alternative autophagy in the present embodiment may not involve the molecular mechanisms of autophagy.

The alternative autophagy activator in the present embodiment may be an alternative autophagy activator that does not activate autophagy.

Intracellular substances are degraded as alternative autophagy is activated, and intracellular metabolism is carried out as the degraded substances are then recycled. In addition, the accumulation of abnormal proteins can be prevented or immune function can be maintained by eliminating foreign invaders. Hence, by the activation of alternative autophagy, functions such as maintaining homeostasis in the body and eliminating abnormal molecules are ameliorated, and thus the condition of all cells and organs in the body can be ameliorated. Therefore, it is possible to prevent various diseases and infections and ameliorate their symptoms. Specifically, the activation of alternative autophagy brings about effects such as regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and/or improvement of germ cell quality.

As an embodiment of the present invention, an alternative autophagy activator is provided which contains at least one selected from the group consisting of a coffee ground extract, a coffee extract, a black pepper extract, a Japanese red pine extract, a juniper berry extract, a turmeric extract, and an apple polyphenol extract and is used for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.

Alternative autophagy may be induced by the fusion of lysosomal proteins with autophagosomes independently of Atg5, Atg7, and/or LC3, and thus as an embodiment of the present invention, an alternative autophagy activator is provided which activates alternative autophagy as at least one selected from the group consisting of a coffee ground extract, a coffee extract, a black pepper extract, a Japanese red pine extract, a juniper berry extract, a turmeric extract, and an apple polyphenol extract promotes fusion of a lysosomal protein with an autophagosome independently of Atg5, Atg7, and/or LC3, and is used for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.

Specifically, the alternative autophagy activator in the present embodiment or the food or beverage composition in the present embodiment contains at least one of the extracts, and thus can activate alternative autophagy, and each of these can be utilized for the purposes listed in Table 1.

**[Table 1]**

| Extract | Purpose |
|---|---|
| Coffee ground extract | At least one selected from regulation of stomach condition, maintenance of intestinal function, rejuvenation of intestines, amelioration of cognitive function, improvement of memory, removal of brain's waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Dehydrocafestol (DC) | At least one selected from regulation of stomach condition, maintenance of intestinal function, rejuvenation of intestines, amelioration of cognitive function, improvement of memory, removal of brain' s waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Dehydrokahweol (DK) | At least one selected from regulation of stomach condition, maintenance of intestinal function, rejuvenation of intestines, amelioration of cognitive function, improvement of memory, removal of brain" s waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Coffee extract | At least one selected from regulation of stomach condition, maintenance of intestinal function, rejuvenation of intestines, amelioration of cognitive function, improvement of memory, removal of brain' s waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Black pepper extract | At least one selected from regulation of stomach condition, maintenance of intestinal function, rejuvenation of intestines, amelioration of cognitive function, improvement of memory, removal of brain's waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Japanese red pine extract | At least one selected from regulation of stomach condition, maintenance of intestinal function, rejuvenation of intestines, amelioration of cognitive function, improvement of memory, removal of brain's waste products. maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Juniper berry extract | At least one selected from regulation of stomach condition, maintenance of intestinal function, rejuvenation of intestines, amelioration of cognitive function, improvement of memory, removal of brain's waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |
| Turmeric extract | At least one selected from regulation of stomach condition, maintenance of intestinal function, rejuvenation of intestines, amelioration of cognitive function, improvement of memory, removal of brain's waste products, maintenance of healthy liver function, improvement of motor function, maintenance of moistness and elasticity of skin, amelioration of blemishes, wrinkles and/or dullness of skin, amelioration of color, moistness and/or sheen of hair. extension of healthy life expectancy, or improvement of germ cell quality |
| Apple polyphenol extract | At least one selected from regulation of stomach condition, maintenance of intestinal function, rejuvenation of intestines, amelioration of cognitive function, improvement of memory, removal of brain's waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthy life expectancy, or improvement of germ cell quality |

In the present embodiment, the alternative autophagy activator may be a promoter of fusion of a lysosomal protein with an autophagosome, an agent for enhancing the degradative action of alternative autophagy, or an intracellular metabolism promoter.

The activation of alternative autophagy may be evaluated by methods known to those skilled in the art. Examples of the evaluation include evaluation of alternative autophagy activity when autophagosomes fuse with lysosomes, and evaluation of activity using fluorescence intensity or electron microscopy. The measurement of alternative autophagy activity may be measurement of the gene expression level of alternative autophagy-related protein groups or measurement of the protein expression level. Specifically, with reference to Patent Literature 1 and the like, the evaluation can be performed using cells deficient in Atg5, Atg7, and/or LC3 that express a lysosomal protein to which a fluorescent protein is attached. Specifically, the evaluation can be performed using the fluorescence intensity generated when alternative autophagy is induced and lysosomal proteins aggregate. Generated fluorescence intensity may be converted into a numerical value and used for the evaluation. With reference to Patent Literature 1 and the like, in a case where the fluorescence intensity due to the aggregation of lysosomal proteins is greater when the alternative autophagy activator of the present embodiment is administered compared to when the alternative autophagy activator of the present embodiment is not administered, it can be evaluated that alternative autophagy is activated. For example, in a case where the fluorescence intensity is increased by 10%, 20%, 30%, 40%, 50%, 60%, 70%, 80%, 90%, 100%, 200%, 300%, 400%, or 500% or more, it can be evaluated that alternative autophagy is activated. Preferably, it can be evaluated that alternative autophagy is activated in a case where the fluorescence intensity is increased by 30% or more.

The alternative autophagy activator can exert its function, for example, by being administered directly or indirectly to an organ or cell in the body.

The dosage form of the alternative autophagy activator may be any dosage form as long as it can exert its function, and the alternative autophagy activator may be administered, for example, in a dosage form such as an oral preparation such as a tablet, a capsule, a granule, a fine granule, a powder, a liquid, a syrup, a chewable tablet, or a lozenge, an ointment, a gel, a cream, an injection, a sublingual, an inhalant, or a suppository. The alternative autophagy activator can be produced using conventionally known additives to be prepared in these dosage forms, and although not particularly limited thereto, for example, excipients, binders, diluents, disintegrants, buffers, thickeners, stabilizers, emulsifiers, dispersants, suspending agents, and/or preservatives known in the art may be used if necessary.

The dose and frequency of administration of the alternative autophagy activator may be appropriately determined depending on various situations such as the route of administration and the age, weight, and symptoms of the administration subject.

The subject to which the alternative autophagy activator is administered is preferably mammals, more preferably humans, monkeys, cats, pigs, horses, cows, mice, rats, guinea pigs, dogs, and rabbits, and still more preferably humans.

As an embodiment of the present invention, a food, beverage, feed, cosmetic, pharmaceutical, quasi-drug product, or the like that contains the alternative autophagy activator in the present embodiment is provided.

The food, beverage, feed, cosmetic, pharmaceutical, quasi-drug product, or the like may be in the same dosage forms as those described as dosage forms of the alternative autophagy activator.

As another embodiment of the present invention, a food or beverage composition for activating alternative autophagy, which contains at least one selected from the group consisting of a coffee ground extract, a coffee extract, a black pepper extract, a Japanese red pine extract, a juniper berry extract, a turmeric extract, and an apple polyphenol extract.

The food or beverage composition can be, for example, in a form suitable for the aspect of use, and is not particularly limited, but may be, for example, in a dosage form such as an oral preparation such as a tablet, a capsule, a granule, a fine granule, a powder, a liquid, a syrup, a chewable tablet, or a lozenge, an ointment, a gel, a cream, an injection, a sublingual, an inhalant, or a suppository. The food or beverage composition is not particularly limited, but can be, for example, in an edible form such as a solid, liquid, granule, particle, powder, capsule, cream, paste, or jelly form. The food or beverage composition may also be coated with one or more layers, if necessary, for example, when prepared in a capsule form. The food or beverage composition can be produced using conventionally known additives to be prepared in these dosage forms or forms, and although not particularly limited thereto, for example, excipients, binders, diluents, disintegrants, buffers, thickeners, stabilizers, emulsifiers, dispersants, suspending agents, and/or preservatives known in the food industry may be used if necessary.

The food or beverage composition is not particularly limited, but may be, for example, a health food, a food labeled with claimed function, a food for specified health uses, a food with nutrient function claims, a health supplement, or a supplement.

Examples of the food or beverage composition include foods, beverages, seasonings, and food additives, specific examples of the foods include confectionery, dairy products, bread, noodles, rice, soups, and prepared foods, and specific examples of the beverages include milk beverages, soft drinks, fruit juice beverages, carbonated beverages, sports drinks, energy drinks, and alcoholic beverages.

If necessary, coloring agents, flavoring agents, sweeteners, bittering agents, acidulants, preservatives, antioxidants, pH adjusters, thickening stabilizers and/or the like may be added to the food or beverage composition.

The food or beverage composition may be provided after being heat sterilized, or may be provided in a sealed form in a bag or container. When provided, the food or beverage composition may be labeled with at least one indication selected from the group consisting of "regulation of stomach condition", "maintenance of intestinal function", "rejuvenation of intestines", "amelioration of cognitive function", "improvement of memory", "removal of brain waste products", "maintenance of healthy liver function", "improvement of motor function", "maintenance of moistness and elasticity of skin", "amelioration of blemishes, wrinkles and/or dullness of skin", "amelioration of color, moistness and/or sheen of hair", "extension of healthspan", and "improvement of germ cell quality".

As another embodiment of the present invention, the food or beverage composition in the present embodiment may be a food or beverage composition that activates alternative autophagy by promoting fusion of a lysosomal protein with an autophagosome independently of Atg5, Atg7, and/or LC3.

The food or beverage composition in the present embodiment is capable of activating alternative autophagy as the food or beverage composition contains at least one selected from the group consisting of a coffee ground extract, a coffee extract, a black pepper extract, a Japanese red pine extract, a juniper berry extract, a turmeric extract, and an apple polyphenol extract, and thus brings about at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.

Hence, as the present embodiment, a food or beverage composition for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality is also provided which contains at least one selected from the group consisting of a coffee ground extract, a coffee extract, a black pepper extract, a Japanese red pine extract, a juniper berry extract, a turmeric extract, and an apple polyphenol extract.

The food or beverage composition in the present embodiment may exhibit at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy, promotion of fusion of a lysosomal protein with an autophagosome independently of Atg5, Atg7, and/or LC3, or activation of alternative autophagy due to promotion of fusion of a lysosomal protein with an autophagosome independently of Atg5, Atg7, and/or LC3 by at least one selected from the group consisting of a coffee ground extract, a coffee extract, a black pepper extract, a Japanese red pine extract, a juniper berry extract, a turmeric extract, and an apple polyphenol extract.

Furthermore, the food or beverage composition in the present embodiment contains at least one of the extracts and thus can activate alternative autophagy, and each of food or beverage compositions containing at least one of the extracts brings about the effects described in Table 1 above. Hence, as the present embodiment, a food or beverage composition bringing about the effects described in Table 1 above is also provided which contains at least one selected from the group consisting of a coffee ground extract, a coffee extract, a black pepper extract, a Japanese red pine extract, a juniper berry extract, a turmeric extract, and an apple polyphenol extract.

The food or beverage composition can exert its alternative autophagy activating action by being orally ingested.

### Examples

Hereinafter, the present invention will be more specifically described with reference to specific examples. The present invention is not limited to this.

### (1) Preparation of sample for alternative autophagy activity measurement

### (Black pepper extract)

A pepper extract (piperine, HJK-3458) from Inabata Koryo Co., Ltd., BioPerine from Sabinsa Japan Corporation, a black pepper extract powder (95%) from VIDYA JAPAN K.K., a black pepper extraction powder (GIH-95) from GOA International, and an in-house extracted black pepper ethanol extract were used.

### (Japanese red pine extract)

Whole dried Japanese red pine was crushed into powder, 30 mL of 99.5% ethanol was added to 3.0 g of this powder, and stirring was performed at room temperature for 30 minutes to perform extraction. Thereafter, the residue was removed by a conventional method, and the extract was then dried to obtain 0.116 g of a dried product.

### (Juniper berry extract)

Whole dried juniper berries were crushed into powder, 30 mL of 99.5% ethanol was added to 3.0 g of this powder, and stirring was performed at room temperature for 30 minutes to perform extraction. Thereafter, the residue was removed by a conventional method, and the extract was then dried to obtain 0.948 g of a dried product.

### (Turmeric extract)

Curcumin C3 Complex (registered trademark) from Sabinsa Japan Corporation was used.

### (Coffee ground pressing)

The grounds generated when coffee beans (after milling) obtained by blending and roasting Arabica beans and Robusta beans were extracted with hot water were dried using a dryer at 80°C for 30 hours to obtain coffee extraction grounds having a moisture value of 3.8%.

Using a roasting kettle, 10 kg of the coffee extraction grounds were heated to 100°C, and after heating, pressing was performed using an oil press (H-54, HANDER OIL MACHINERY) to obtain oil. At this time, pressing was performed three times to recover a total of 700 g of oil. The recovered oil was filtered using No2 filter paper to finally obtain 425 g of coffee oil (coffee ground pressing).

### (Fraction of coffee ground pressing)

Into a separatory funnel having a capacity of 100 mL, 5.0 g of coffee oil was weighed and put, and 20 mL of hexane was added, and mixing was thoroughly performed. Furthermore, 10 mL of methanol was added and mixed. After it was visually confirmed that the mixture was separated into two layers, the methanol layer was recovered. Furthermore, 5 mL of methanol was added and mixed, and the methanol layer was similarly recovered. This process was repeated two times. The recovered methanol layer was placed in a separatory funnel having a capacity of 100 mL, 0.5 mL of distilled water was added and mixed. The hexane layer was separated, and the methanol layer was recovered in a weighed eggplant flask having a capacity of 50 mL. The methanol layer was dried to obtain 0.42 g of a dried product.

### (80% v/v ethanol extract of coffee grounds)

To 100 g of coffee extraction grounds, 900 mL of 80% v/v ethanol was added. Extraction was carried out for 1 hour using a stirrer in a thermostatic bath at 30°C, and then filtration was carried out using a Buchner funnel lined with No2 filter paper. The permeate after filtration was recovered and added into an eggplant flask, and ethanol was removed by performing concentration under reduced pressure to obtain a concentrate. The concentrate was frozen at -80°C, and the frozen concentrate was dried using a freeze dryer.

### (99.5% v/v ethanol extraction of coffee grounds)

To 100 g of coffee extraction grounds, 900 mL of 99.5% v/v ethanol was added. Extraction was carried out for 1 hour using a stirrer in a thermostatic bath at 30°C, and filtration was carried out using a Buchner funnel lined with No2 filter paper. The permeate after filtration was recovered and added into an eggplant flask, and ethanol was removed by performing concentration under reduced pressure to obtain a concentrate.

### (Acetone extract of coffee grounds)

To 200 g of coffee extraction grounds, 800 mL of acetone was added. Extraction was carried out for 1 hour using a stirrer in a thermostatic bath at 30°C, and filtration was carried out using a Buchner funnel lined with No2 filter paper. The permeate after filtration was recovered and added into an eggplant flask, and acetone was removed by performing concentration under reduced pressure to obtain a concentrate.

### (Dehydrocafestol (DC) and dehydrokahweol (DK))

The grounds generated when coffee beans (after milling) obtained by blending and roasting Arabica beans and Robusta beans were extracted with hot water were dried using a dryer at 80°C for 16 hours to obtain coffee extraction grounds having a moisture value of 10% or less.

The dried coffee extraction grounds were heated to 100°C using a roasting kettle, and after heating, pressing was performed using an oil press (H-54, HANDER OIL MACHINERY). At this time, pressing was performed three times to recover a total of 700 g of oil. The recovered oil was filtered using No2 filter paper to finally obtain 425 g of coffee ground pressed oil.

To a separatory funnel, 5 g of the coffee ground pressed oil was added, hexane in an amount four times the amount of the coffee ground pressed oil was added thereto, mixing was performed, and then methanol in an amount two times the amount of the coffee ground pressed oil was added and mixed. After the mixture was left to still stand for a while and it was confirmed that the mixture was separated into two layers, the methanol layer was recovered. Methanol in an amount equal to that of the coffee ground pressed oil was again added to and mixed with the recovered methanol layer. The procedure was repeated two times to obtain a methanol layer. Distilled water in an amount 1/10 times the amount of the coffee ground pressed oil was added to and mixed with this methanol layer, the mixture was left to still stand for separation, and then the methanol layer was recovered. The recovered methanol layer was concentrated to dryness to obtain 0.42 g of a dried product. The dried product was fractionated using a medium pressure preparative liquid chromatograph (Yamazen Corporation, EPCLC-W-Prep 2XY) under the following conditions. Using Universal Premium ODS-SM 30 µm 120 A, 2L size (3 × 20 cm, 51 g) (Yamazen Corporation) as a column and ultrapure water, acetonitrile, and isopropanol as mobile phases, linear gradient elution was performed at a flow velocity of 20 mL/min from ultrapure water-acetonitrile (95:5) to 100% acetonitrile for 40 minutes, and then isopropanol was allowed to flow for 12 minutes to perform fractionation every 10 mL. The fraction eluted between 37 and 45 minutes was concentrated to dryness. Isolation of DC and DK from the dried product was performed using a preparative HPLC (Shimadzu Corporation, Prominence) under the following conditions. Using SunFire 5 µm (19 × 150 mm) (Waters) as a column and ultrapure water and acetonitrile as mobile phases, ultrapure water-acetonitrile (60:40) was allowed to flow at a column temperature of 30°C and a flow velocity of 17 mL/min for 160 minutes, and the peak eluted between 116 and 126 minutes (component A) and the peak eluted between 128 and 141 minutes (component B) were fractionated to obtain 5 mg and 14 mg of concentrated dry products of components A and B, respectively.

The components A and B isolated as described above were subjected to NMR and mass spectrometry. For NMR measurements, AVANCE NEO 400 MHz instrument (Bruker) was used, mass spectrometry was performed using Vanquish Flex liquid chromatograph (Thermo Fisher Scientific) and Thermo Fisher Scientific OrbiTrap Explorers 240 mass spectrometer (Thermo Fisher Scientific) to acquire the following data, and the component A was identified as dehydrokahweol (DK) and the component B was identified as dehydrocafestol (DC).

The concentrated dry products of components A and B were designated as in-house purified DK and DC, respectively. For evaluation of alternative autophagy activity, a commercially available DC was also used as a sample.

The results of NMR and mass spectrometry of DK are presented below.
¹H-NMR (DMSO-d6, 400 MHz, corrected with solvent): δ (ppm) 7.50 (1H, d, J = 1.5 Hz, H19), 6.45 (1H, brs, H18), 6.29 (1H, d, J = 10.0 Hz, H2), 5.93 (1H, d, J = 10.1 Hz, H1), 5.34 (1H, s, H15), 4.65 (1H, brt, -OH, H17), 3.99 (2H, s, H17), 2.54 - 2.50 (2H, overlapped, H5, 13), 2.01 (1H, d, J = 10.2 Hz, H14a), 1.86 (1H, m, H6a), 1.78 - 1.33 (9H, overlapped, H6b, 7a, 7b, 9, 11a, 11b, 12a, 12b, 14b), 0.94 (3H, s, H20)
¹³C-NMR (DMSO-d6, 100 MHz, corrected with solvent): δ (ppm) = 149.37 (C3), 147.22 (C16), 141.72 (C19), 138.60 (C1), 133.60 (C15), 121.43 (C4), 115.03 (C2), 109.05 (C18), 59.05 (C17), 48.62 (C8), 44.43 (C14), 43.72 (C5), 41.23 (C10), 40.36 (C13), 40.00 (C9), 37.26 (C7), 24.86 (C12), 20.62 (C6), 19.02 (C11), 15.18 (C20)
ESI MS (m/z) C₂₀H₂₃O ([M+H-H2O]+), theoretical value 279.1743, measured value 279.1742; C₂₀H₂₅O₂ ([M+H]+), theoretical value 297.1849, measured value 297.1847

The results of NMR and mass spectrometry of DC are presented below.
¹H-NMR (DMSO-d6, 400 MHz, corrected with solvent): δ (ppm) = 7.39 (1H, d, J = 1.4 Hz, H19), 6.29 (1H, d, J = 1.7 Hz, H18), 5.32 (1H, s, H15), 4.62 (1H, brt, -OH, H17), 3.99 (2H, brs, H17), 2.57 - 2.55 (2H, m, H2), 2.21 (1H, dd, J = 12.5, 1.9 Hz, H5), 2.11 (1H, d, J = 10.2 Hz, H14a), 2.03 (1H, dt, J = 12.8, 4.2 Hz, H1a), 1.79 - 1.33 (10H, overlapped, H6a, 6b, 7a, 7b, 11a, 11b, 12, 14b), 1.24 - 1.17 (1H, m, H1b), 1.14 (1H, d, J = 8.4 Hz, H9), 0.79 (3H, s, H20)
¹³C-NMR (DMSO-d6, 100 MHz, corrected with solvent): δ (ppm) = 148.00 (C3), 147.04 (C16), 140.84 (C19), 133.97 (C15), 119.88 (C4), 108.50 (C18), 59.08 (C17), 48.24 (C8), 44.48 (C14), 43.62 (C9), 43.47 (C5), 40.48 (C13), 38.02 (C10), 37.81 (C7), 35.17 (C1), 25.01 (C12), 21.56 (C6), 20.20 (C2), 18.95 (C11), 12.93 (C20)
ESI MS (m/z) C₂₀H₂₅O ([M+H-H2O]+), theoretical value 281.1900, measured value 281.1899; C₂₀H₂₇O₂ ([M+H]+), theoretical value 299.2006, measured value 299.2005

### (DC and DK high content ground extract)

To 200 g (as dry weight) of the coffee extraction grounds obtained above of which the moisture value was adjusted to 2.5%, 70% v/v ethanol was added to make up a total volume of 1 L. Stirring was performed at 50°C for 2 hours at 250 rpm using Three-One Motor (BL-600, manufactured by Shinto Scientific Co., Ltd.). After 2 hours of extraction, filtration was carried out using No2 filter paper, and the filtrate and the extraction grounds on the filter paper were recovered. The recovered extraction grounds on the filter paper were mixed with 70% v/v EtOH to make up a total volume of 1 L, and then extraction was carried out again at 50°C for 1 hour while stirring was performed. After 1 hour of extraction, filtration was carried out using No2 filter paper, and the filtrate was recovered. The first and second filtrates were collected and concentrated using an evaporator until EtOH was removed, and the concentrate was freeze-dried. After freeze drying, 7.9 g of extract was obtained. The present extract was designated as a DC and DK high content ground extract.

### (Apple polyphenol extract)

Apple Phenon (registered trademark) (manufactured in China) sold by BGG Japan was used.

### (2) Evaluation of alternative autophagy activity

DMEM (containing 4.5 g/L glucose) medium (Sigma-Aldrich) containing 10% FBS (Sigma Aldrich), 1 mM sodium pyruvate solution (Nacalai Tesque), 1 mM HEPES buffer (Nacalai Tesque), 0.1 mM non-essential amino acid solution (FUJIFILM Wako Pure Chemical), 0.1 mM MEM Vitamin solution (Invitrogen), 100 U/mL penicillin-100 µg/mL streptomycin (Nacalai Tesque), and 0.05 mM 2-mercaptoethanol (Bio-Rad) was used.

With reference to Patent Literature 1 (WO 2013/118842), ATG5-KO/GFP-Lamp1 was prepared as follows.

Construction was performed by the method described in Mizushima, N. et al. Dissection of autophagosome formation using Apg5-deficient mouse embryonic stem cells. J. Cell Biol. 152, 657 - 668 (2001). A gene expressing SV40 T antigen was introduced into mouse embryonic fibroblasts (MEFs) established from ATG5-deficient C57/B6J mice at embryonic day 14.5 by electroporation using the Nucleofector system (Amaxa) for immortalization. Furthermore, a gene expressing a fusion protein of Lamp1 and GFP (also referred to as "GFP-Lamp1") was introduced using a retrovirus constructed by introducing an MSCV-Lamp1-GFP (zeocin-resistant) vector into plat E cells, which were retrovirus packaging cells. Then, cells with high expression of GFP-Lamp1 were cloned and prepared as ATG5-KO/GFP-Lamp1.

ATG5-KO/GFP-Lamp1 was seeded at a concentration of 4.0 × 10⁴ cells/mL onto a 384-well plate (Greiner) by 30 µL and cultured in an incubator at 37°C and 5% CO₂ for 24 hours. Furthermore, the prepared coffee ground extract, the coffee ground pressing, the dehydrocafestol (DC), the dehydrokahweol (DK), the DC and DK high content ground extract, the black pepper extract, the Japanese red pine extract, the juniper berry extract, the turmeric extract, and the apple polyphenol extract were each added. After 24 hours, 4% paraformaldehyde-phosphate buffer solution (FUJIFILM Wako Pure Chemical) was added by 12 µL, and the mixture was left to still stand at room temperature for 15 minutes. After the culture was washed with HBSS (FUJIFILM Wako Pure Chemical), HBSS mixed with 30 µL of Hoechst (Invitrogen) diluted 2000-fold was added to each well by 30 µL, and the mixture was left to still stand at room temperature for 15 minutes.

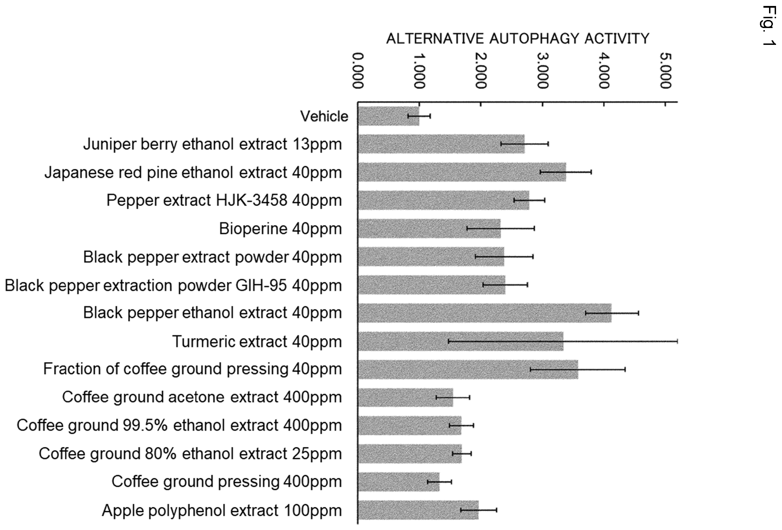

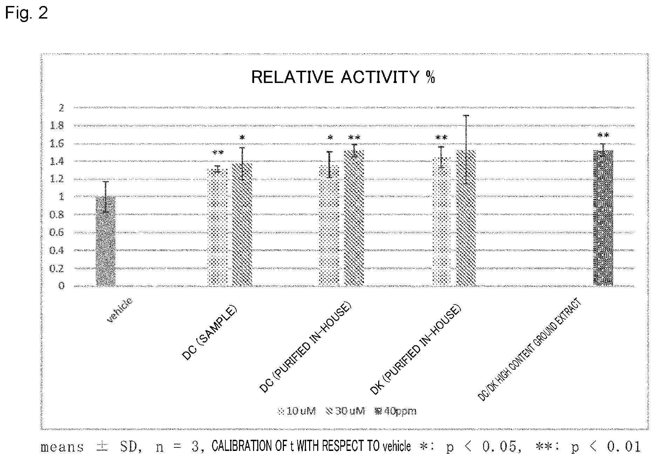

Thereafter, microscopic images were acquired by photographing using a confocal quantitative image cytometer CQ1 (Yokogawa Electric Corporation). With reference to Patent Literature 1, the integrated value of the fluorescence intensity of GFP-Lamp1 fluorescent granules per cell was output from the microscopic image, the value was divided by that of the vehicle group, and the value thus acquired was used as an index to evaluate alternative autophagy activity. The results are presented in Table 2 and Figure 1. The results for DC, DK, and DC and DK high content ground extract are illustrated in Figure 2.

Similar experiments were conducted using available extracts of the extract disclosed in Patent Literature 2, and the results revealed that these extracts were not sufficiently effective in activating alternative autophagy (results not disclosed).

**[Table 2]**

| | |
|---|---|
| Vehicle | 1.000 |
| Juniper berry ethanol extract 13ppm | 2.709 |
| Japanese red pine ethanol extract 40ppm | 3.378 |
| Pepper extract HJK-3458 40ppm | 2.785 |
| BioPerine 40ppm | 2.323 |
| Black pepper extract powder 40ppm | 2.378 |
| Black pepper extraction powder GIH-95 40ppm | 2.395 |
| Black pepper ethanol extract 40ppm | 4.129 |
| Turmeric extract 40ppm | 3.336 |
| Fraction of coffee ground pressing 40ppm | 3.575 |
| Coffee ground acetone extract 400ppm | 1.547 |
| Coffee ground 99.5% ethanol extract 400ppm | 1.687 |
| Coffee ground 80% ethanol extract 25ppm | 1.693 |
| Coffee ground pressing 400ppm | 1.329 |
| Apple polyphenol extract 100ppm | 1.968 |

From the results in Table 2 and Figures 1 and 2, it has been confirmed that a coffee ground extract, a coffee ground pressing, dehydrocafestol (DC), dehydrokahweol (DK), a DC and DK high content ground extract, a black pepper extract, a Japanese red pine extract, a juniper berry extract, a turmeric extract, and an apple polyphenol extract have the function of activating alternative autophagy.

### <Example>

The results of investigating the changes in DC and DK contents in the coffee extraction grounds obtained above under the respective drying conditions are presented in Table 3. The DC and DK contents per extract when freeze-dried grounds were extracted with 75% v/v EtOH were regarded as 100%, and the DC + DK contents (%) were calculated based on this.

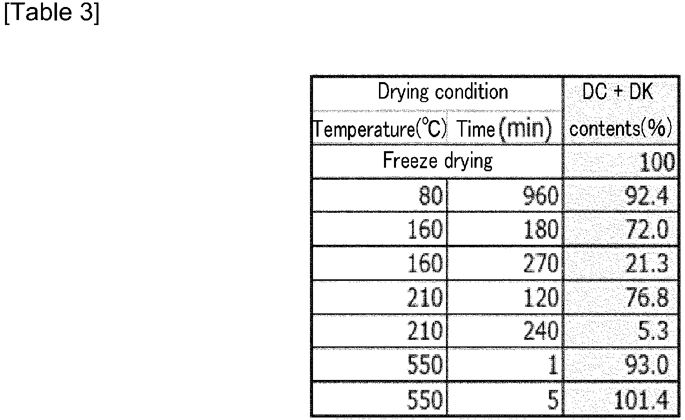

## Claims

1. An alternative autophagy activator comprising at least one selected from the group consisting of a coffee extract and/or a coffee ground extract, a black pepper extract, a Japanese red pine extract, a juniper berry extract, a turmeric extract, and an apple polyphenol extract.

2. The alternative autophagy activator according to claim 1, which activates alternative autophagy as at least one selected from the group consisting of a coffee extract and/or a coffee ground extract, a black pepper extract, a Japanese red pine extract, a juniper berry extract, a turmeric extract, and an apple polyphenol extract promotes fusion of a lysosomal protein with an autophagosome independently of Atg5, Atg7, and/or LC3.

3. The alternative autophagy activator according to claim 1 or 2, which is used for at least one purpose selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality.

4. A food or beverage composition for activating alternative autophagy, the food or beverage composition comprising at least one selected from the group consisting of a coffee extract and/or a coffee ground extract, a black pepper extract, a Japanese red pine extract, a juniper berry extract, a turmeric extract, and an apple polyphenol extract.

5. A food or beverage composition for at least one selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality, the food or beverage composition comprising at least one selected from the group consisting of a coffee extract and/or a coffee ground extract, a black pepper extract, a Japanese red pine extract, a juniper berry extract, a turmeric extract, and an apple polyphenol extract.

6. The food or beverage composition according to claim 5, which exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality by activation of alternative autophagy by at least one selected from the group consisting of a coffee extract and/or a coffee ground extract, a black pepper extract, a Japanese red pine extract, a juniper berry extract, a turmeric extract, and an apple polyphenol extract.

7. The food or beverage composition according to claim 5 or 6, which exhibits at least one action selected from the group consisting of regulation of stomach condition, maintenance of intestinal function, rejuvenation of intestines, amelioration of cognitive function, improvement of memory, removal of brain waste products, maintenance of healthy liver function, improvement of motor function, maintenance of moistness and elasticity of skin, amelioration of blemishes, wrinkles and/or dullness of skin, amelioration of color, moistness and/or sheen of hair, extension of healthspan, and improvement of germ cell quality as at least one selected from the group consisting of a coffee extract and/or a coffee ground extract, a black pepper extract, a Japanese red pine extract, a juniper berry extract, a turmeric extract, and an apple polyphenol extract promotes fusion of a lysosomal protein with an autophagosome independently of Atg5, Atg7, and/or LC3.
